# EUROPEAN PATENT SPECIFICATION

(11) **EP 4 552 116 B1**
(45) Date of publication and mention of the grant of the patent: **10.06.2026**
(21) Application number: 23720192.6
(22) Date of filing: 24.04.2023
(51) Int. Cl.: G09F 23/00, G09F 23/06, G09F 19/00, G09F 3/00, C11D 17/00, E03D 13/00, G09B 19/00

(54) **ARTICLE FOR USE IN A TOILET OR URINAL**
ARTIKEL ZUR VERWENDUNG IN EINER TOILETTE ODER EINEM URINAL
ARTICLE DESTINÉ À ÊTRE UTILISÉ DANS UN WC OU UN URINOIR

(30) Priority: 05.07.2022 EP 22183043
(43) Date of publication of application: 14.05.2025
(73) Proprietor: Latisus B.V., 2408 AV Alphen aan den Rijn (NL)
(72) Inventor: HAVENGA, Menzo Jans Emko, 2408 AV Alphen aan den Rijn (NL); DUIJVELAAR, Lennart Jan, 2408 AV Alphen aan den Rijn (NL)
(74) Representative: van Dam, Vincent
(86) International application number: PCT/NL2023/050217
(87) International publication number: WO 2024/010443

(56) References cited:
- EP-A1- 2 014 758
- CA-A1- 2 315 137
- JP-A- 2002 143 036
- KR-A- 20160 113 774
- US-A- 5 285 540
- US-A1- 2020 279 504
- US-B1- 6 183 850
- US-B1- 6 815 403

## Description

The present invention relates to an article for use in a toilet or urinal. The invention also relates to a toilet or urinal, comprising said article. The invention further relates to a method for entertaining users of a toilet or urinal, in which said article is applied. The invention also relates to a method for manufacturing said article. The invention also relates to a computer readable medium, comprising instructions for 3D printing said article.

### Introduction

The present invention finds its application in toilets and urinals. It is known that disintegrating blocks containing various components can be used for cleaning, disinfecting and/or deodorizing toilets or urinals. Such disintegrating blocks generally are placed on the bottom surface of a toilet or urinal, or are placed in a holder of some sort and then put under the rim of the toilet bowl or urinal. Once put into place, such a block slowly releases active ingredients and disintegrates into the water. The present invention provides an article that can also be placed or thrown on the bottom surface of a toilet or urinal or be placed on a support device placed in a bottom part of a toilet or urinal, but which serves a fundamentally different purpose.

US 6 183 850 B1 discloses a toilet target device comprising a water-dispersible foam polymer target floating in a toilet bowl or resting in a potty or urinal is provided. Also provide is a toilet target containing a fragrance, a method for preparing a toilet target, and a method for promoting potty training for children.

CA 2 315 137 A1 discloses the use of polyalkylene glycols for improving the moisture stability of cleaning tablets and to certain cleaning tablets with improved moisture stability which contain polyalkylene glycols.

US 6 815 403 B1 discloses a toilet drain composition and method of using for use in disinfecting and decalcifying, wherein the toilet drain composition comprises a phosphoric acid derivative admixed with paradichlorobenzene and a diluent.

KR 2016 0113774 A discloses an artistic soap having multiple advertisement surfaces.

US 5 285 540 A discloses a toilet training system and method which includes a plurality of sheets with patterns adapted to dissolve when contacted with urine, at least one chart for recording the child's use of a toilet, instruction for positioning one of the sheets in the toilet such that the patterned sheet is contacted by the child's urine and therefore dissolves, and instructions for recording the child's toilet training progress on the provided chart.

US 2020/279504 A1 discloses a floating device acting as a target for enhancing the accuracy of a urine stream.

EP 2 014 758 A1 discloses a wedge-shaped cleaner for cleaning and fragrancing toilets, comprising a transparent phase and an advertisement carrier, which is a molded part produced by thermal deformation and is completely enclosed by transparent phase and/or a cleaning agent phase.

### Summary of the invention

The present invention relates in one aspect to an article for use in a toilet or urinal, wherein the article comprises a visual trigger which motivates a user of the toilet or urinal to urinate onto said article, and wherein the composition of the article comprises a filler component selected from lactose and mannitol or a combination thereof; and a binder component selected from a cellulose derivative, a starch derivative or a polyvinyl pyrrolidone compound, or a combination thereof; and wherein the article has dimensions designed to allow the user to have the article disintegrated in a single pee when urinating onto the article, wherein a single pee is defined as urinating 175-400 ml urine during 5 - 30 seconds; wherein said article is a 3D printed article comprising a picture or text as a visual trigger printed throughout at least part of the thickness of said article.

In a second aspect the present invention relates to a toilet or urinal, comprising said article placed on a surface intended to directly receive a urine stream from a user of said toilet or urinal.

In accordance with the invention the visual trigger of the article triggers a user of the toilet or urinal to pee onto the article and the composition of said article is designed to allow the user to have the article disintegrated in a single pee when urinating onto the article.

The fact that the article can completely dissolve in a single pee motivates the user to aim or direct his urine flow such to realize complete disintegration of the article. This makes urinating fun or even enables a competition when used in public toilets, and thus brings about an entertaining effect. The purpose of the invention is therefore in one aspect to provide people, more specifically males in the age group 15-55, with a fun moment allowing them to relief themselves on a personally (dis)liked character/ person/ team/ effigy or other.

The invention also relates to a method for entertaining users of a toilet or urinal as defined in claim 10.

The inventors have found that the article according to the invention can very suitably be manufactured by means of 3D printing. Therefore, the invention relates in another aspect to a method for manufacturing the article according the invention, comprising 3D printing said article. In view of this the invention relates in a still further aspect to a computer readable medium, comprising instructions for 3D printing the article according to the invention in accordance with said method for manufacturing. In this regard 3D printing may add an extra dimension to the entertaining effect mentioned above, because 3D printing allows to obtain an article with multiple layers with images that may differ per layer or layers without image alternated with layers that have images. This way a revealing effect can be achieved, so that, for instance, when a user aims and urinates on the article, initially a certain image or text is revealed which was hidden from sight before the user started urinating, after which, upon further urinating the full article fully disintegrates.

### Short description of the drawings

Fig.1 shows a picture of a 3D printed tablet in accordance with the invention with a visual trigger on the top side (Fig.1A, showing a view from the top side of the tablet) which motivates a user of the toilet or urinal to urinate onto the crosshair on the top side of the tablet, and a bottom side opposite to the top side containing a text for promotional purposes (Fig. 1B showing a view from the bottom side of the tablet).
Fig.2 schematically shows side views of tablets with variations with regard to the design of the tablet edges. In a first design the tablet has sharp edges (Fig. 2A). In a second, third and fourth design as shown in Fig. 2 B, C and D, respectively the edges are rounded to various extents.
Fig.3 A-D show pictures of 3D printed tablets of the designs of Fig. 2A-D, respectively. The upper panel shows a picture of the top side of the tablets. The lower panel shows a picture of the side edges of the tablet.

### Detailed description of the invention.

The present invention is based on the finding that an article as defined in claim 1, containing a visual trigger which motivates a user of a toilet or urinal to urinate onto said article, and wherein the composition of the article is designed to allow the user to have the article disintegrated in a single pee when urinating onto the article can be used to entertain the user of the toilet or urinal. Another point of view regarding the use of the article according to the invention relates to the use of the article in a marketing method, which is also part of the present disclosure. This method comprises the step of handing out the article according to the invention to a user while collecting personal data of the user. In an embodiment thereof articles according to the invention are packaged as single units which can be provided to a user while collecting user data (location, frequency of use, time). For instance, an article can be provided on the condition that a user of the article submits data via an app of QR like technique. The collected user data represent a value for advertisers. Apart from the visual trigger of the article, additional incentives to persuade a user to submit data may be implemented. Additional incentives in this respect may include prizes associated with "winning" articles, which may for instance be articles that show a unique colour, pattern and/or message when urinated upon. In this respect, the articles according to the invention can also be used in a contest or lottery method which is also part of the present disclosure. In such a lottery or contest method a prize can be won when a user has obtained one or more of the aforementioned winning articles.

The article can be used in a urinal or a toilet. This can be a private or public toilet. The invention is in particular suitable for application in a toilet area at large events such as festivals or at large venues or buildings with many toilet users. These can be traditional toilet bowls, urinals, gutters or any other place or means where people urinate. As mentioned above the invention aims suitably at males in the age group of 15-55 years. This is because these will be the average persons using a urinal, and therefore the article's composition and specifications are preferably designed such that an average male between 15 and 55 is capable of disintegrating the article according to the invention by peeing on it. Nevertheless the invention is also suitable for male persons of any other age which are able to urinate while standing up or female or trans persons. In this case the use of a female urination device may be preferred so as to enable urinating while standing up. In this respect the disintegration time of the article may also be adapted to the intended user.

The article according to the invention may be provided in a toilet area, for instance in a bowl and may be disposed into the toilet or urinal by the user. Alternatively, the articles can be provided in a dispenser automatically or manually disposing an article according to the invention before a user starts urinating.

In order to prevent that an article starts disintegrating before a person starts urinating on it and also because positioning an article in a toilet or urinal can be difficult, a small support device may be provided, for instance comprising a suction cup to attach the support device on the bottom or back side of the toilet or urinal. The support device may comprise a flat platform with an optional protruding rim. This allows to position the article in the toilet or urinal in an optimal and stable position so that disintegration only takes place when a person urinates on it and not by flushing water, for instance.

In one embodiment the article may be shaped such that the article itself forms the visual trigger. The shape in this respect may be any design that motivates a user of a toilet or urinal to urinate onto said article. For instance the article can be provided in the shape of a person, an animal, a building or a word or phrase that invokes an urge to urinate onto the article.

In a preferred embodiment it is not the shape of the article itself that form the visual trigger, but the visual trigger is a picture or text comprised in said article. The visual trigger may for instance be selected from an image selected among popular figures, a logo or brand name selected from the field of entertainment, cartoons, politics, sports, science etc. The article in this embodiment can be provided the shape of a block or tablet, for instance in a square, cylinder, bead, or flat disk, for instance a circular disk. These shapes allow high throughput production against low costs. In order to realize a shaped article and for purposes of production, it is preferred that the article in accordance with the invention is composed of a solid material.

A flat tablet is preferred. This allows minimal use of material, while the visual trigger can be maximally exposed to the user of the toilet or urinal. The flat tablet can suitably be a flat disc. Without limitation such a flat disc may have a square, rectangular, elliptic, or circular shape or any other shape. To have the article disintegrated in a single pee when urinating onto the article and to keep an entertaining function, the dimensions of the tablet should preferably be chosen such that it takes about 5-30 s of peeing on it to have the tablet completely disintegrated. It is even more preferred that the tablet completely dissolves withing between 5 and 15 seconds of peeing on it to ensure a good dissolving result within the duration of a single pee. These parameters apply in particular to male persons between 15 and 55. A tablet with a diameter size between 10 and 50 mm and a thickness between 2 and 6 mm, such as a diameter size of approximately 40 and a thickness of 3 to 5 mm, such as 4 mm, would for instance be suitable for these purposes because the limited diameter allows the tablet to be fully contacted with a urine stream produced by a user. The thickness of the tablet affects the disintegration behaviour. The thicker the tablet, the longer it takes for a tablet to disintegrate.

The picture or text extends through at least a part of the thickness of the tablet. This way the visual trigger is longer visible to the user urinating on the tablet, which is advantageous for the motivation of the user to urinate onto the tablet. In view of this it, is even more preferred that the picture or text extends through the major part of the thickness of the tablet, even more preferably fully through the thickness of the tablet.

For marketing purposes the tablet may comprise a top side containing said visual trigger which motivates a user of the toilet or urinal to urinate onto the top side of the tablet, and a bottom side opposite to the top side containing a text or picture for promotional purposes. Such a text or picture for promotional purposes may for instance be a company or product name or trademark. When urinated upon, the article should fully disintegrate in a single pee when urinating onto the article. An average pee usually comprises a flow of 175-400 ml urine during 5 - 30 seconds. It is therefore preferred that a single pee is defined as urinating 175-400 ml urine during 5 - 30 seconds.

Alternatively, the requirement that the article should fully disintegrate in a single pee when urinating onto the article may be defined by way of the urine flow simulation test described in the examples section below which comprises the steps of heating water to 37 °C, placing the article in a sieve underneath an outlet from which water flows to simulate urinary flow, wherein water is flown over the article at a rate of 10 ml/s, which corresponds to the lower limits of the urinary flow of an average adult male. Under these circumstances the article should fully disintegrate within 5 - 30 seconds.

As already indicated above, it is even more preferred that the article completely dissolves within between 5 and 15 seconds of peeing on it to ensure that the article dissolves within the duration of a single pee. Disintegration or dissolving the article should be such that 95% of the users urinating onto the article see the article fully disintegrate or dissolve as a result of their own effort and not from pre-existing wetness or flushing water in the toilet or urinal. The composition and/or dimensions of the article may therefore be adapted to the type of urinal or toilet, depending on the presence of flushing water.

The tablet must be sized and shaped in such way that it is easy to throw/position it in most urinals.

In an embodiment wherein a tablet is thrown into a toilet or urinal it may be preferred that a tablet does not end up face down, i.e. with the visual trigger image down. In this case a straight forward coin shape may be extended to include hyperbolic edges and/or slight hyperbole mid points.

In some embodiments it may be preferred that the tablet has a floating capacity, so that is floats on pre-existing water/urine in a toilet or urinal. To improve floating capability the bottom may be designed more curved than the top.

The visual trigger may be applied onto or throughout the article in various ways. For instance an article may comprise at least two layers of which at least one top layer contains the visual trigger. This top layer may then be glued to the bottom layer(s), for instance by heat-glueing. The top layer(s) may be provided as a single sheet with an image or as multiple layers of material with the same image to provide said image throughout the thickness of the article. For instance, in one embodiment the article may comprise multiple layers, including at least one inner layer comprising a visual trigger initially hidden from sight by a covering layer which is closer to the surface of the article compared to said inner layer or at the surface, wherein said visual trigger can be revealed upon urinating onto said covering layer.

In accordance with the invention the article with a visual trigger is 3D printed. 3D printing techniques allow an image to be printed throughout at least part of the thickness of an article, such as in particular a tablet, i.e. from bottom to top. For instance, Powder Bed Printing (PBP), including Binder Jetting, Multi Jet Fusion and Selective Laser Sintering (SLS) or a combination thereof as exemplary Powder Bed Printing methods may be considered in the context of the present invention as they can be used to create highly porous tablets that can dissolve quickly, also depending on the choice of materials.

As mentioned above, a visual trigger in the form of an image is present not only on the outer surface of the tablet but also at least partially throughout the thickness of tablet, in order to assure that the image is visible for longer periods of time during the disintegration of the tablet, possibly even allowing changes in the tablet to take place. By tuning the laser settings SLS is often capable of creating different shades of brown during the consolidation process. This is caused by the heat generated that in turn can lead to a local colouring of the material similar to the browning of food products when exposed to heat in an oven. In fact, SLS can be used to create images in that way using shades of browning. It would also be possible this way to create the image (or different images) on each layer of the printed object.

In addition to the disintegration speed a further preferred requirement is the presence of a full/multicolour image on the tablet. In view of this printing may suitably take place using a Powder Bed Printing (PBP) device. PBP is more preferred because in contrast to SLS it can be used to create full-colour 3D objects using a range of (typically white) powder materials in combination with coloured printing inks. The printing process is similar to the creation of colour images using an inkjet document printer. The material requirements for PBP process can be split into two: those pertaining to the powders and those pertaining to the printing liquids ("inks"). Besides the requirements for the process, the specific envisioned application will pose additional requirements for the materials. This will be explained below, with reference to suitable materials and compositions.

A very suitable PBP printing approach involves Binder Jetting. In Binder Jetting, a printing ink is selectively deposited onto a powder bed, bonding these areas together to form a solid article, such as a tablet, one layer at a time. When the 3D printer has nozzles to deposit printing ink, it is possible to create full-color articles. In this approach, typically first a thin layer of powder is spread over the build platform. Then, a carriage with inkjet nozzles passes over the bed, selectively depositing droplets of a binding agent (glue) that bond the powder particles together. In full-colour Binder Jetting, the coloured ink is also deposited during this step. The size of each drop is approximately 50 µm in diameter, so good resolution can be achieved. When the layer is complete, the build platform moves downwards and the blade re-coats the surface. This is repeated until the article is complete. After printing, the article is encapsulated in the powder and is left to cure and gain strength. Then the article is removed from the powder bin and any unbound, excess powder is removed using pressurized air.

With respect to the powders the requirements are typically that the powders must be free-flowing and of a particle size distribution that matches the layer thicknesses that are being used.

Although printing processes can be carried out under controlled atmosphere, ideally the powders are non-hygroscopic and non-static, nor should they have melting points close to the operating temperatures of the printer (around room temperature) as meeting these specifications will prevent issues with stickiness and powder deposition on printing heads, depositors, etc.

Naturally, in order to achieve consolidation of the powders into a solid 3D object the individual powder particles must be stuck together by means of the effect of the printing liquid being deposited. The following routes are preferred options for consolidation.

In a first route the printing liquid can be a glue-type material that makes the particles stick together, without the particles needing to have any sticking characteristics themselves. This route does not really pose any requirements on the powder materials, other than having to be wettable by the printed glue droplets.

In a second route the printing liquid interacts with the powder particles (or at least with one type of particulate material if a blend is used) in that it at least partially dissolves the particles, resulting in liquid bridges between the individual particles in which the liquid contains dissolved material. The amount of liquid added will determine the amount of binding and in turn the time it takes for the tablet to dissolve. Upon drying the particles are stuck together by the now solidified previously dissolved material, resulting in a solid object. The degree of dissolution of the powder material in the printing liquid can vary, and the creation of sticky outer surfaces of particles can be enough to make the material stick together. As mentioned, not all the powder material needs to be soluble, as blends can be used that contain filler-type powders and glue-type powders. Naturally enough of the glue-type powder must be present to make the entire 3D object stick together. This route does pose requirements on the material, specifically on the glue-type powders in that they need to be soluble to some degree in the printing liquid that is being used, allowing them to stick together upon drying.

The type of inks that can be used in the powder printing process may depend on the printing heads that are being employed, as each printing head poses specific requirements with respect to the viscosity and surface tension of the liquids. Good results with 3D printing tablets have for instance been achieved with inks based on dyes dissolved in a water/ethanol mixture, for instance in a 70/30 volume ratio and using inks in a percentage of 15-30 wt.% with respect to the weight of the powders (binder and filler).

When looking at the different types of printing heads that can be used in the context of the present invention these can be divided into two types of single nozzle valves, specifically passive/non-piezo and piezo-based, and multi-nozzle valve systems. Each of these have their own advantages and disadvantages. The passive single nozzle valves are relatively cheap but have a limited range of fluid properties they can handle. In contrast, the piezo-based single nozzle systems can handle a much broader range of fluids, but are more expensive. Being single nozzle, both systems however are slow with respect to the deposition of a layer of liquid. With regard to speed, multi-nozzle systems are preferred as they can be used for so-called "single pass" printing (i.e. deposition of a whole layer of droplets in a single pass).

In a suitable embodiment the tablets are manufactured by 3D printing to have rounded edges instead of sharp edges. This may have advantageous effects on the appearance of a printed tablet.

The materials used for manufacturing the article according to the invention are preferably environmentally safe. Preferably the materials are food grade materials. This way, the article does not harm a person accidently mistaking the article for a piece of candy instead of an article intended for use in a toilet or urinal.

A powder formulation for 3D printing in accordance with the abovementioned second route is typically composed of two basic components, a (polymer) binder and a filler. The binder component may comprise a single binding agent or multiple binding agents. Likewise the filler component may comprise a single filler agent or multiple filler agents. The terms "binder" and "filler" in the context of the present invention may thus refer to a single binder or filler agent or to multiple binder or filler agents. The binder interacts with the print fluid in that it can (at least partially) dissolve in order to bind the rest of the material after drying. The polymer binder fraction in the powder mixtures is typically around 10-30 wt.%, with the rest being filler. Both polymer binder powder and filler powder have to have the right particle size (range) (typically a size D50 of 50-60 µm), allowing it to be successfully used in the PBP process. Additionally, the particle size will also influence the final resolution and surface roughness of the printed object. In order to improve the disintegration behaviour it is possible to add a disintegrant (microcrystalline cellulose, sodium starch glycolate, etc.) similar to the use of such compounds in pharmaceutical tablets. Alternatively methods to change the disintegration behaviour of the tablet are: changing the ratio of binder: filler (a higher amount of binder results in longer disintegration time); changing the printing liquid amounts or other printing parameters (a higher amount of printing liquid results in longer disintegration time); changing the way in which the printing liquid is distributed, including linespacing, droplets/mm; changing layer height; changing print fluid; changing the design of the tablet; alone or in any combination.

Fillers are selected from lactose and mannitol, or a combination thereof. These are food grade and can be used in a 3D printing process, and in particular a PBP 3D printing process.

Polymer binders may are selected from a cellulose derivative, a starch derivative or a polyvinyl pyrrolidone compound, or a combination thereof. Suitable examples of starch derivatives are pregelatinized starch, starch, dextrin, maltodextrin, and the like. Suitable examples of cellulose derivatives include hydroxypropyl cellulose, methyl cellulose and the like. Suitable polyvinyl pyrrolidone compounds include polyvinyl pyrrolidone, vinylpyrrolidone-vinyl acetate copolymers etc. These compounds are food grade and can be used in a 3D printing process, in particular a PBP process.

In view of this, the composition of the article of the invention comprises a first component selected from lactose and mannitol, or a combination thereof, and a second component selected from a cellulose derivative, a starch derivative or a polyvinyl pyrrolidone compound or a combination thereof.

The powder composition for 3D printing may also be dependent on the print fluid (ink) used. For instance for a water based printing fluid the combinations pregelatinized starch / lactose, polyethylene glycol / mannitol, sugar / lactose, maltodextrin / lactose, or sugar / maltodextrin may be suitable. On the other hand for a water/ethanol based printing fluid the combinations pregelatinized starch / lactose, vinylpyrrolidone-vinyl acetate copolymer / mannitol, polyvinyl pyrrolidone/ mannitol, hydroxypropyl cellulose / mannitol, or polyethylene glycol / sugar may be suitable.

Good results with regard to appearance - as well as image appearance and overall appearance of a printed tablet - and disintegration time were observed when lactose was used as a first component in combination with a cellulose and/or starch derivative. In a preferred embodiment the composition of the article therefore comprises lactose as said first component and a component selected from a cellulose derivative and a starch derivative as said second component. Herein it is preferred that the binder component comprises a cellulose derivative and a starch derivative.

Good balance between appearance, disintegration time was obtained with tablets comprising lactose and starch, in particular pregelatinized starch. The combination of lactose and starch performs well and is minimally affected by the composition of the urine to which it is exposed. It is noted that the combination of lactose and starch results in a particularly satisfying disintegration behaviour. Namely, during the first seconds of peeing an article with these lactose and starch as main ingredients does not visibly change and only starts to visibly disintegrate after this first short period of time. On the other hand, within 10-30 seconds articles of this composition will be fully disintegrated so that no residues are left behind in the toilet of urinal. The apparent initial stability before disintegration starts to be visible to the person urinating on it increases the entertaining effect of the article, because said person has to put some effort in successfully peeing the article away, while the reward (disappearance of the article) is usually guaranteed.

It is therefore, preferred that the composition of the article comprises lactose as said first component and a starch, preferably pregelatinized starch as said second component. With regard to these embodiments preferred ratios between first and second component may range from 90:10 to 70:30 (weight: weight). In a preferred embodiment the composition of the article comprises two main ingredients: lactose as filler in an amount of 70-80 wt.% and starch as a binder in an amount of 20-30 wt.%, with preferred particle sizes of D50 50-60. Higher amounts of starch result in longer disintegration times of the article and vice versa. The above amounts result in a good balance between printability and disintegration time.

In a highly preferred embodiment, the article comprises 70 - 80 weight % of lactose, 15 - 25 weight % of starch and 5 - 10 weight % of cellulose based on the total weight of lactose, cellulose and starch. Herein the particle sizes D50 of the binder and filler preferably range between 50 and 100 µm, preferably between 65-90µm, such as between 70-85 µm, with a maximum size of 125 µm. With these components, ratios and sizes the articles have a very good balance between appearance and disintegration time. In particular, in these ratios the lactose component has been found to contribute to a good printing bed when the articles are produced by printing, the starch component contributed to obtaining a fast drying, firm product, with good visual appearance, with little release of powder during handling and transport, and the cellulose component on its turn contributes to uniform disintegration behaviour, so that the product disintegrates layer wise in an erosive behaviour. This way the visual triggers may remain visible until the article has fully or nearly fully disintegrated, which enhances the fun effect of the article.

Apart from fillers and binders, further compounds may also be included in the article according to the invention, including without limitation flavours or aromas for odour and to render the article edible, surface active compounds and/or disinfectants for a cleaning, or any other additive.

### Examples

The following examples show various embodiments of articles for use according to the invention and exemplary methods for manufacturing said articles. The present examples are intended to illustrate the invention and are not intended to limit the scope of the invention.

An investigation was conducted with regard to manufacturing tablets for use in a toilet or urinal, the tablets having visual triggers motivating a user of the toilet or urinal to urinate onto said article, and wherein the composition of the article is designed to allow the user to have the article disintegrated in a single pee when urinating onto the article. Within the context of the present invention the term disintegration may encompass dissolving the material of the article in liquid and/or disintegration into particles small enough to be flushed through a toilet. Full disintegration implies that no tablet residue is left sticking on the surface supporting the tablets.

Tablets were envisaged with a flat surface showing disintegration ideally between 5 and 30 seconds when exposed to a simulated urinary flow.

### Test 1

Small circular tablets with a diameter size of 15 mm and a thickness of 4 mm were 3D printed using a Powder Bed Printing (PBP) device (ProJet CJP 660 Pro) based on various compositions and printing specifications. The following materials were used:
- Amylogum CLS, starch, D50= 70 µm, Mw low, AVEBE,
- Respitose SV003, lactose,<45 µm 0-40/26%, <63 µm 40-70/54%, <100 µm 90-100/97%, DFE Pharma
- HPC SLFP, Hydroxy propyl cellulose, D50=80-110 µm, Mw 100.000, Nisso
- Kollidon K25, Polyvinylpyrrolidone, Sigma, product code: 02286
- Kollidon VA64, vinylpyrrolidone-vinyl acetate copolymers, <50 µm max 35%, >250 µm max 7%, Mw 45.000-75.000, BASF
- Maltodextrin DE19, Aldrich, product code: 419699
- Mannitol, product code: 25311.366, VWR
- Methyl cellulose, sigma-Aldrich, product code:M0262
- Prejel PA5 PH, starch, D10=10 µm, D50=65 µm, D90=156 µm, DFE Pharma

### Printing conditions:

- Tablets were printed with 70/30 & 50/50 v/v demi water/ethanol (+0.05 w/v% blue dye)
- Dye: patent blue V for microscopy (Sigma-Aldrich 20262-76-4)
- Dissolve 0.02 g of dye in 28 mL of demiwater, then add 12 mL of EtOH. Filter (millex 5 µm) the solution, then degas for 5 min in an ultrasonic bath using the degas setting.
- In the formulations ratios of 80/20 were used between two powder components. The ratios are typically based on prior experience with 3D printing of pharmaceutical tablets. Other ratios may also be successful for the purposes of the present invention.
- Linespacing 0.30, 0.35, 0.40, 0.45, 0.50 mm. The linespacing range reflects the amount of liquid (i.e. ink) deposited per surface area. Higher line spacings indicate bigger distances between droplet lines that are deposited and hence a lower amount of liquid per surface area.
- Tablets diameter = 15 mm, height = 4 mm, layer thickness = 0.4 mm
- Tablets removed from powder bed 30 s after print is finished
- Drying in oven 50 °C overnight (optional).

### Urinary flow simulation test

In order to simulate a person urinating on the tablets water was heated in a water bath to 37 °C (body temperature). Tablets were placed in a sieve underneath the tube from which water could be expelled, simulating the urinary flow. Using a pump, water was flown over the sample at a rate of 10 mL/s, which corresponds to the lower limits of the urinary flow of an average adult male. Movies were made of the disintegration/dissolution behavior of the various tablets. The required disintegration/dissolution time was reported, as well as the general behavior of the tablet when wetted. Tests results were confirmed on small scale by a urinating person.

**Table 1: Performance of various formulations of tablets in a urinary flow simulation test.**

| Formulation nr. | Formulation | Ratio components w/w | Printing liquid ratio water/ethanol (v/v) | Line spacing (mm) | Disintegration |
|---|---|---|---|---|---|
| 1 | Lactose SV003 / amylogum | 80/20 | 50/50 | 0.30 | Start disintegration at 6 s, complete disintegration at 16 s |
| 2 | Lactose SV003 / amylogum | 80/20 | 70/30 | 0.35 | Start disintegration at 14 s, complete disintegration at 20 s |
| 3 | Lactose SV003 /amylogum | 80/20 | 70/30 | 0.40 | Start disintegration at 10 s, complete disintegration at 15 s |
| 4 | Lactose SV003 / amylogum | 80/20 | 70/30 | 0.40 | Start disintegration at 14 s, complete disintegration at 20 s |
| 5 | Lactose SV003 /HCP SLFP | 80/20 | 50/50 | 0.40 | Start disintegration at 3 s, complete disintegration at 19 s |
| 6 | Lactose SV003 / Kollidon 25 | 80/20 | 70/30 | 0.30 | Start disintegration at 7 s, complete disintegration at 15 s |
| 7 | Lactose SV003 / Kollidon 25 | 80/20 | 50/50 | 0.30 | Start disintegration at 12 s, complete disintegration at 20 s |
| 8 | Lactose SV003 /methyl cellulose | 80/20 | 50/50 | 0.35 | Start disintegration at 4 s, complete disintegration at 30 s |
| 9 | Lactose SV003 /methyl cellulose | 80/20 | 70/30 | 0.40 | Start disintegration at 10 s, complete disintegration at 15 s |
| 10 | Lactose SV003 /methyl cellulose | 80/20 | 50/50 | 0.40 | Start disintegration at 3 s, complete disintegration at 14 s |
| 11 | Lactose SV 003/ prejel | 80/20 | 70/30 | 0.35 | Start disintegration at 3 s, complete disintegration at 30 s |
| 12 | Mannitol / Kollidon K25 | 80/20 | 50/50 | 0.30 | Start disintegration at 10 s, complete disintegration at 16 s |
| 13 | Mannitol Kollidon VA64 | 80/20 | 50/50 | 0.30 | Start disintegration at 10 s, complete disintegration at 15 s |
| 14 | Mannitol / Kollidon VA64 | 80/20 | 50/50 | 0.35 | Start disintegration at 3 s, complete disintegration at 19 s |
| 15 | Mannitol / HPC SLFP | 80/20 | 50/50 | 0.40 | Start disintegration at 20 s, complete disintegration at 30 s |
| 16 | Mannitol / HPC SLFP | 80/20 | 70/30 | 0.45 | Complete disintegration between 15 - 30 s. |
| 17 | Mannitol / HPC SLFP | 80/20 | 70/30 | 0.50 | Complete disintegration between 15 - 30 s. |

### Appearance

The tablets produced with the formulations listed in Table 1 were assessed based on their visual appearance wherein tablets with high visibility of print and smooth surface were ranked from 1 - 5, wherein #1 has the best appearance. The results are shown in Table 2 below:

**Table 2: Top 5 appearance score of tablets.**

| Ranking | Formulation no. | Formulation |
|---|---|---|
| #1 | 11 | Lactose SV003/ prejel |
| #2 | 2 | Lactose SV003/ amylogum |
| #3 | 8 | Lactose SV003/ methyl cellulose |
| #4 | 15 | Mannitol / HPC SLFP |
| #5 | 5 | Lactose SV003 /HCP SLFP |

### Dual image printing

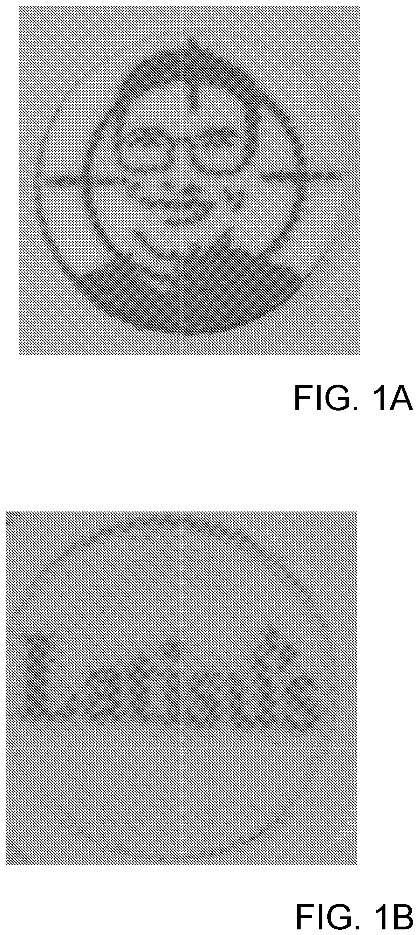

With the composition of formulation no. 11 a dual printed tablet with a diameter of 40 mm and a thickness of 4 mm was printed as shown in Fig. 1 with a visual trigger on the top side (Fig.1A) which motivates a user of the toilet or urinal to urinate onto the top side of the tablet, and a bottom side opposite to the top side containing a text for promotional purposes (Fig. 1B). To achieve this, tablets were printed with the face/cross-hairs image in the top 6/7 layers and the Latisus logo in the bottom 2 layers, with a blank intermediate layer between the image and the text to avoid that the top image can often be seen through the bottom layers.

### Shape of the tablet

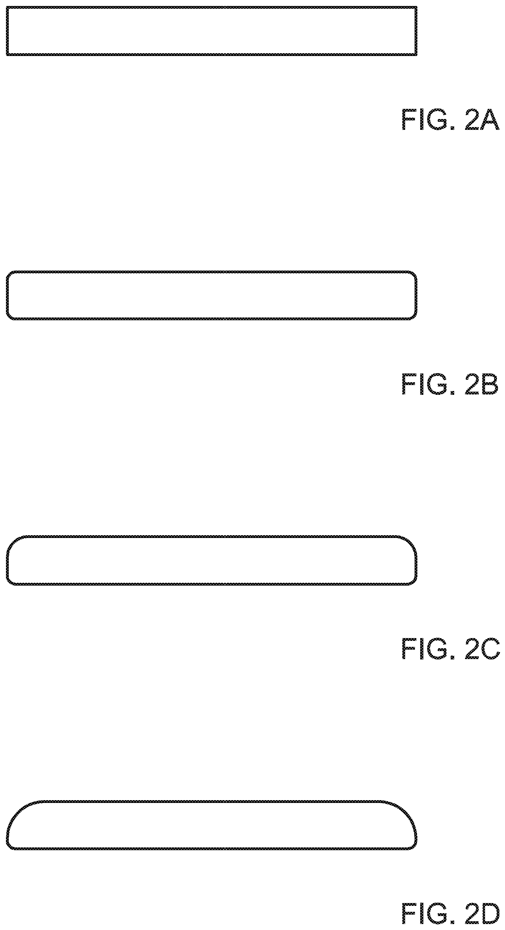

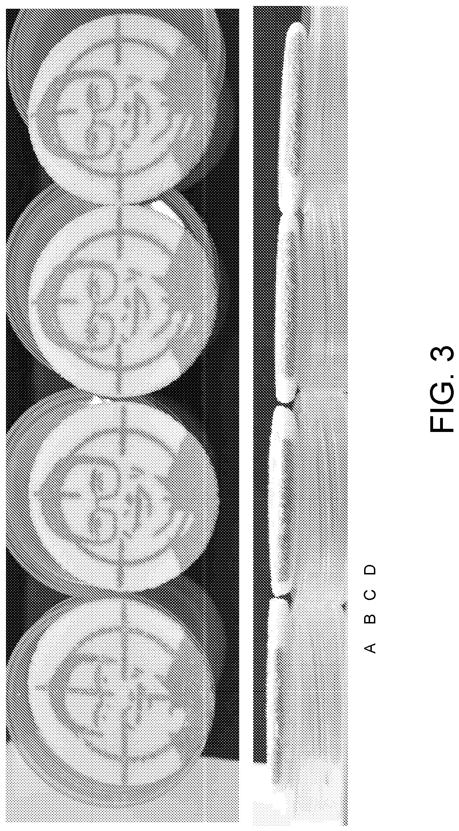

The inventors have found that the resolution of the visual trigger can be enhanced by designing the shape of the tablet. In a first design the tablet has sharp edges (Fig. 2A). In a second, third and fourth design as shown in Fig. 2 B, C and D, respectively the edges are rounded. Fig. 2B shows identical top and bottom rounding. Fig. 2C shows exaggerated rounding of the top. Fig. 2D shows strongly exaggerated rounding of the top. Fig. 3 shows tablets printed with formulation no.11 with sharp edges (Fig.3A), and according to the abovementioned second, third and fourth design (Fig. 3B-D). Similar results were obtained with formulations 2 (lactose SV003/ amylogum) and 8 (lactose SV003/ methyl cellulose). It is clear from Fig. 3 that rounding the edges of the tablet results in improved appearance in terms of visibility of print of the tablet.

### Test 2

Tablets were 3D printed using the Binder Jetting approach. The following materials were used:
- Inks based on a dyes dissolved in a water/ethanol mixture in a 70/30 volume ratio.
- Lactose filler (Respitose SV003 (monohydrate lactose) D50 particle size = 61 µm.
- Starch binder (Amylogum CLS) D50 particle size = 70 µm, Mw low.

Printer ink was used in an amount of 15-30 wt.% with respect to the powder portion. Performance of various formulations of tablets was tested in a urinary flow simulation test as described above. Tests results were confirmed on small scale by a urinating person. The results are shown in table 2 below:

**Table 2: Performance of various formulations of Binder Jetting produced tablets in a urinary flow simulation test.**

| Formulation | Composition | Tablet thickness | Disintegration |
|---|---|---|---|
| 18 | 80/20 lactose/starch | 4mm | Disintegrates after 4-6 s, complete disintegration shortly after that. |
| 19 | 75/25 lactose starch | | Start disintegrating after 6-8 s. Complete disintegration at 11 seconds |
| 20 | 70/30 lactose starch | | Disintegrates after 4-6 s, complete disintegration shortly after that. |

It is particular noted in relation to the lactose/starch based tablets (both PBP printed and Binder Jetting printed) that during the first seconds of peeing the tablets do not visibly change and only start to visibly disintegrate after this short period of time. On the other hand, within 10-20 seconds tablets of this composition are fully disintegrated so that no residues are left behind in the toilet of urinal. The initial apparent stability of the tablet before disintegration starts to be visible to the person urinating on it increases the entertaining effect of the tablet, because said person has to put some effort in successfully peeing the tablet away, while the reward (disappearance of the tablet) is guaranteed.

### Test 3

Tablets were 3D printed using the Binder Jetting approach. The following materials were used:
- Inks based on a dyes dissolved in a water/ethanol mixture in a 70/30 volume ratio.
- Lactose filler (Pharmatose 125 M (monohydrate lactose) D50 particle size = 80 µm.
- Starch binder (Amylogum CLS) D50 particle size = 70 µm, Mw low.
- Cellulose binder (HPC SSL, hydroxypropylcellulose, D50 = 85 µm.

Tests were performed with binder and filler portions containing 70 - 80 wt.% lactose, 15- 25 wt.% starch and 5-10 wt.% cellulose (powder) Tablets were made using 4 - 6 g powder and using 1.0-1.5 ml ink per tablet. Performance of tablets was tested in a urinary flow simulation test as described above and it was confirmed on small scale by a urinating person that the tablets produced disintegrated completely over a period of 8 to 15 seconds. In addition, the tablets had good visual appearance, with little release of powder during handling and transport, were firm and fast-drying, and had uniform disintegration behaviour, so that the tablet disintegrates layer wise in an erosive behaviour so that visual triggers may remain visible until the tablet had fully or nearly fully disintegrated, thus enhancing the fun effect of the tablet.

## Claims

1. Article for use in a toilet or urinal, wherein the article comprises a visual trigger which motivates a user of the toilet or urinal to urinate onto said article, and wherein the composition of the article comprises
a binder component selected from a cellulose derivative, a starch derivative or a polyvinyl pyrrolidone compound, or a combination thereof;
wherein the article has dimensions designed to allow the user to have the article disintegrated in a single pee when urinating onto the article, wherein a single pee is defined as urinating 175-400 ml urine during 5 - 30 seconds;
**characterized in that** the composition of the article comprises a filler component selected from lactose and mannitol or a combination thereof, and **in that** said article is a 3D printed article comprising a picture or text as a visual trigger printed throughout at least part of the thickness of said article.

2. Article according to claim 1, wherein the binder component comprises a cellulose derivative and a starch derivative.

3. Article according to any of the previous claims, wherein the article comprises 70 - 80 weight % of lactose, 15 - 25 weight % of starch and 5 - 10 weight % of cellulose based on the total weight of lactose, cellulose and starch.

4. Article according to any of the previous claims, wherein the particle sizes D50 of the binder and filler ranges between 50 and 100 µm, preferably between 65 - 90 µm, such as between 70 - 85 µm.

5. Article according to claim 4, wherein said article is a tablet comprising said picture or text.

6. Article according to claim 5, wherein the tablet comprises a top side containing said visual trigger which motivates a user of the toilet or urinal to urinate onto the top side of the tablet, and a bottom side opposite to the top side containing a text or picture for promotional purposes.

7. Article according to any of the previous claims, wherein the article comprises multiple layers, including at least one inner layer comprising a visual trigger initially hidden from sight by a covering layer which is closer to the surface of the article compared to said inner layer or at the surface, wherein said visual trigger can be revealed upon urinating onto said covering layer.

8. Article according to any of the previous claims wherein said visual trigger is based on an ink based on a dye dissolved in a water/ethanol mixture, preferably in a 70/30 volume ratio and wherein the ink was used in a percentage of 15-30 wt.% with respect to the weight of the binder and filler.

9. Toilet or urinal, comprising the article according to any of the previous claims placed on a support device intended to directly receive a urine stream from a user of said toilet or urinal.

10. Method for entertaining users of a toilet or urinal, comprising:
providing an article according to any of the claims 1 to 8 placed on a surface intended to directly receive a urine stream from a user of said toilet or urinal.

11. Method for manufacturing the article according to any of the claims 1 to 8, comprising 3D printing the article according to any of the claims 1 to 8.

12. Method according to claim 11, wherein 3D printing comprises using a Powder Bed Printing (PBP) device, preferably wherein the method involves Binder Jetting.

13. Method according to any of the claims 11 to 12, comprising applying ink in a percentage of 15-30 wt.% with respect to the weight of the binder and filler.

14. Computer readable medium, comprising instructions for 3D printing the article according to any of the claims 1-8 in accordance with the method according to any of the claims 11 to 13.

## Patentansprüche

1. Artikel zur Verwendung in einer Toilette oder einem Urinal, wobei der Artikel einen visuellen Auslöser umfasst, der einen Benutzer der Toilette oder des Urinals dazu veranlasst, auf den Artikel zu urinieren, und wobei die Zusammensetzung des Artikels eine Bindemittelkomponente umfasst, die aus einem Cellulosederivat, einem Stärkederivat oder einer Polyvinylpyrrolidonverbindung oder einer Kombination davon ausgewählt ist;
wobei der Artikel Abmessungen hat, die darauf ausgelegt sind, es dem Benutzer zu ermöglichen, den Artikel bei einer einzigen Blasenentleerung zum Auflösen zu bringen, wenn er auf den Artikel uriniert, wobei eine einzige Blasenentleerung als das Urinieren von 175-400 ml Urin innerhalb von 5 bis 30 Sekunden definiert ist;
**dadurch gekennzeichnet, dass** die Zusammensetzung des Artikels eine Füllstoffkomponente umfasst, die aus Lactose und Mannitol oder einer Kombination davon ausgewählt ist, und dadurch, dass
der Artikel ein 3D-gedruckter Artikel ist, der ein Bild oder einen Text als visuellen Auslöser umfasst, der durch zumindest einen Teil der Dicke des Artikels hindurch gedruckt ist.

2. Artikel nach Anspruch 1, wobei die Bindemittelkomponente ein Cellulosederivat und ein Stärkederivat umfasst.

3. Artikel nach einem der vorhergehenden Ansprüche, wobei der Artikel 70-80 Gew.-% Lactose, 15-25 Gew.-% Stärke und 5-10 Gew.-% Cellulose bezogen auf das Gesamtgewicht von Lactose, Cellulose und Stärke umfasst.

4. Artikel nach einem der vorhergehenden Ansprüche, wobei die Partikelgröße D50 des Bindemittels und des Füllstoffs im Bereich zwischen 50 und 100 µm, vorzugsweise zwischen 65 und 90 µm, beispielsweise zwischen 70 und 85 µm, liegt.

5. Artikel nach Anspruch 4, wobei der Artikel eine Tablette ist, die das Bild oder den Text umfasst.

6. Artikel nach Anspruch 5, wobei die Tablette eine Oberseite, die den visuellen Auslöser enthält, der einen Benutzer der Toilette oder des Urinals zum Urinieren auf die Oberseite der Tablette veranlasst, und eine der Oberseite entgegengesetzte Unterseite umfasst, die einen Text oder ein Bild zu Werbezwecken enthält.

7. Artikel nach einem der vorhergehenden Ansprüche, wobei der Artikel mehrere Schichten umfasst, einschließlich mindestens einer inneren Schicht, die einen visuellen Auslöser umfasst, der anfänglich durch eine Deckschicht vor dem Blick verborgen ist, die näher an der Oberfläche des Artikels als die innere Schicht oder an der Oberfläche liegt, wobei der visuelle Auslöser beim Urinieren auf die Deckschicht freigelegt werden kann.

8. Artikel nach einem der vorhergehenden Ansprüche, wobei der visuelle Auslöser auf einer Tinte basiert, die auf einem Farbstoff basiert, der in einem Wasser-Ethanol-Gemisch gelöst ist, vorzugsweise in einem Volumenverhältnis von 70/30, und wobei die Tinte in einem Prozentsatz von 15-30 Gew.-% bezogen auf das Gewicht des Bindemittels und des Füllstoffs verwendet wurde.

9. Toilette oder Urinal, umfassend den Artikel nach einem der vorhergehenden Ansprüche, der auf einer Tragvorrichtung platziert ist, die dazu bestimmt ist, einen Urinstrahl von einem Benutzer der Toilette oder des Urinals direkt zu empfangen.

10. Verfahren zum Unterhalten von Benutzern einer Toilette oder eines Urinals, umfassend:
Bereitstellen eines Artikels nach einem der Ansprüche 1 bis 8, der auf einer Oberfläche platziert ist, die dazu bestimmt ist, einen Urinstrahl von einem Benutzer der Toilette oder des Urinals direkt zu empfangen.

11. Verfahren zur Herstellung des Artikels nach einem der Ansprüche 1 bis 8, umfassend das 3D-Drucken des Artikels nach einem der Ansprüche 1 bis 8.

12. Verfahren nach Anspruch 11, wobei das 3D-Drucken das Verwenden einer Pulverbett-Druck- (PBP) -Vorrichtung umfasst, vorzugsweise wobei das Verfahren Binder Jetting einbegreift.

13. Verfahren nach einem der Ansprüche 11 und 12, umfassend das Aufbringen von Tinte mit einem Prozentsatz von 15-30 Gew.-% bezogen auf das Gewicht des Bindemittels und des Füllstoffs.

14. Computerlesbares Medium, umfassend Anweisungen zum 3D-Drucken des Artikels nach einem der Ansprüche 1 bis 8 gemäß dem Verfahren nach einem der Ansprüche 11 bis 13.

## Revendications

1. - Article destiné à être utilisé dans des toilettes ou un urinoir, l'article comprenant un déclencheur visuel qui incite un utilisateur des toilettes ou de l'urinoir à uriner sur ledit article, et la composition de l'article comprenant un composant liant choisi parmi un dérivé de cellulose, un dérivé d'amidon ou un composé polyvinyl pyrrolidone, ou une combinaison de ceux-ci ;
l'article présentant des dimensions conçues pour permettre à l'utilisateur de le désintégrer en un seul jet lorsqu'il urine sur l'article, un seul jet étant défini comme l'émission de 175 à 400 ml d'urine pendant 5 à 30 secondes ;
**caractérisé par le fait que** la composition de l'article comprend un composant de charge choisi parmi le lactose et le mannitol ou une combinaison de ceux-ci, et **par le fait que** ledit article est un article imprimé en 3D comprenant une image ou un texte en tant que déclencheur visuel imprimé à travers au moins une partie de l'épaisseur dudit article.

2. - Article selon la revendication 1, dans lequel le composant liant comprend un dérivé de cellulose et un dérivé d'amidon.

3. - Article selon l'une quelconque des revendications précédentes, dans lequel l'article comprend 70 à 80 % en poids de lactose, 15 à 25 % en poids d'amidon et 5 à 10 % en poids de cellulose sur la base du poids total de lactose, de cellulose et d'amidon.

4. - Article selon l'une quelconque des revendications précédentes, dans lequel la taille de particule D50 du liant et de la charge se situe dans la plage entre 50 et 100 µm, de préférence entre 65 et 90 µm, par exemple entre 70 et 85 µm.

5. - Article selon la revendication 4, dans lequel ledit article est une tablette comprenant ladite image ou ledit texte.

6. - Article selon la revendication 5, dans lequel la tablette comprend une face supérieure contenant ledit déclencheur visuel qui incite un utilisateur des toilettes ou de l'urinoir à uriner sur la face supérieure de la tablette, et une face inférieure opposée à la face supérieure contenant un texte ou une image à des fins promotionnelles.

7. - Article selon l'une quelconque des revendications précédentes, dans lequel l'article comprend plusieurs couches, dont au moins une couche interne comprenant un déclencheur visuel initialement masqué à la vue par une couche de recouvrement qui est plus proche de la surface de l'article que ladite couche interne ou qui se trouve à la surface, ledit déclencheur visuel pouvant être révélé lors de la miction sur ladite couche de recouvrement.

8. - Article selon l'une quelconque des revendications précédentes, dans lequel ledit déclencheur visuel est à base d'une encre à base d'un colorant dissous dans un mélange eau/éthanol, de préférence dans un rapport en volume de 70/30, et dans lequel l'encre a été utilisée dans un pourcentage de 15 à 30 % en poids par rapport au poids du liant et de la charge.

9. - Toilettes ou urinoir, comprenant l'article selon l'une quelconque des revendications précédentes placé sur un dispositif de support destiné à recevoir directement un jet d'urine provenant d'un utilisateur desdites toilettes ou dudit urinoir.

10. - Procédé destiné à divertir des utilisateurs de toilettes ou d'un urinoir, comprenant : la mise à disposition d'un article selon l'une quelconque des revendications 1 à 8 placé sur une surface destinée à recevoir directement un jet d'urine provenant d'un utilisateur desdites toilettes ou dudit urinoir.

11. - Procédé de fabrication de l'article selon l'une quelconque des revendications 1 à 8, comprenant l'impression 3D de l'article selon l'une quelconque des revendications 1 à 8.

12. - Procédé selon la revendication 11, dans lequel l'impression 3D comprend l'utilisation d'un dispositif d'impression par lit de poudre (PBP), de préférence dans lequel le procédé implique une projection de liant.

13. - Procédé selon l'une quelconque des revendications 11 et 12, comprenant l'application d'encre dans un pourcentage de 15 à 30 % en poids par rapport au poids du liant et de la charge.

14. - Support lisible par ordinateur, comprenant des instructions pour l'impression 3D de l'article selon l'une quelconque des revendications 1 à 8, conformément au procédé selon l'une quelconque des revendications 11 à 13.
